Europäisches Patentamt
European Patent Office
Office européen des brevets

(19)

(11) Publication number: **0 340 837 A1**

(12) # EUROPEAN PATENT APPLICATION

(21) Application number: 89201037.2

(51) Int. Cl.4: A61K 39/21 , C12N 15/00

(22) Date of filing: 21.04.89

The applicant has filed a statement in accordance with Rule 28 (4) EPC (issue of a sample only to an expert). Accession number(s) of the deposit(s): Accession No. 20876, 67669 and 67670.

(30) Priority: 25.04.88 US 185626
25.04.88 US 185711
25.04.88 US 185572
09.12.88 US 282145
09.12.88 US 282146
09.12.88 US 282144

(43) Date of publication of application: 08.11.89 Bulletin 89/45

(84) Designated Contracting States: AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Vlasuk, George P.
786 Hartley Drive
Lansdale Pennsylvania 19446(US)
Inventor: Polokoff, Mark A.
3607 Pinhorn Drive
Bridgewater New Jersey 08807(US)
Inventor: Dixon, Richard A. F.
758 Hartley Drive
Landsdale Pennsylvania 19446(US)
Inventor: Schultz, Loren D.
421 Oak Drive
Harleysville Pennsylvania 19438(US)
Inventor: Hofmann, Kathryn J.
1001 Townsend Circle
Wayne Pennsylvania 19087(US)
Inventor: Silberklang, Melvin
21 Hillside Avenue
Englewood New Jersey 07631(US)
Inventor: Ellis, Ronald W.
1407 Edgevale Road
Overbrook Hills Pennsylvania 19151(US)
Inventor: Emini, Emilio A.
6 Faggs Manor Lane
Paoli Pennsylvania 19301(US)
Inventor: Gerety, Robert J.
1003 Stonebridge Road
Lower Gwynedd Pennsylvania 19002(US)

(74) Representative: Hesketh, Alan, Dr.
European Patent Department Merck & Co., Inc. Terlings Park Eastwick Road
Harlow Essex, CM20 2QR(GB)

(54) **Recombinant GAG precursor of HIV, product, process, and use as aids vaccine.**

(57) The protein yeast recombinant gag precursor of greater than 95% purity or equally pure variant thereof. This protein is myristoylated and therefore different in at least one respect from E. coli-produced recombinant gag precursor. Recombinant gag precursor is alternatively expressed in a recombinant insect eypression system. The purified gag precursor is useful as a vaccine and in commercial assays of AIDS viral protease.

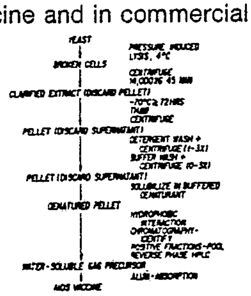

FIG. I

EP 0 340 837 A1

# RECOMBINANT GAG PRECURSOR OF HIV, PRODUCT, PROCESS, AND USE AS AIDS VACCINE

Acquired Immunodeficiency Syndrome (AIDS) is the clinical manifestation of the apparent infection of CD4 helper T-cells by human immunodeficiency virus (HIV), also previously referred to as human T-lymphotropic virus type III (HTLV-III), Lymphoadenopathy-associated virus (LAV), or AIDS-related virus (ARV) (hereinafter collectively "HIV"). AIDS is a transmissible deficiency of cellular immunity characterized by opportunistic infections and certain malignancies. A similar disease, AIDS-related complex (ARC), shares many of the epidemiological features and immune abnormalities with AIDS, and often precedes the clinical manifestations of AIDS.

A vaccine against AIDS and/or ARC is an ideal prophylactic treatment for preventing the delibilitating effects of infection by HIV. Applicants have discovered an immunogen useful for such a vaccine as well as methods of purifying recombinant forms of the immunogen. The immunogen is the gag precursor polypeptide in recombinant form.

Many of the details of the genetic function and virion structure of HIV have not yet been elucidated. However, certain general features have emerged. An RNA virus with a genome totaling about 9 kilobases (kb), its nucleotide sequence contains seven major open reading frames (ORFs) corresponding to the gag, pol and env, sor, tat, art/trs and 3′orf genes. Terminal repeats in the nucleotide sequence are common to many retroviruses such as HIV and are required for viral replication and integration into the host chromosome. More recent discussions on the general nature of HIV genomic structure, replication and regulation are found in Ratner, L. et al. "Human T-Lymphotropic Retroviruses," in O'Brien, S.J. (ed.) Genetic Maps 1987 Cold Spring Harbor 1987 pp. 124-129; Franchini, G. et al. Nature 328, 539 (1987); and Chen, I.S.Y. Cell 47, 1 (1986).

The HIV proteins that initially are translated from the genes gag, pol and env appear as polyprotein fusion products. Subsequent proteolytic processing results in structural proteins of the virus as well as viral enzymes. In some strains of HIV, the gag polyprotein fusion product appears as a p55 gag precursor, which then is proteolytically cleaved by a virus encoded protease into gag p24, gag p17 and gag p15 during the virus life cycle. There is evidence that occasional frame shifting during translation or some other mechanism leads sometimes to a giant gag-pol polyprotein fusion product. In HIV, the enzymatic site that catalyzes proteolytic processing is coded by a stretch of nucleotides located about 250-265 nucleotide bases within pol, downstream and 3′ of the translation stop codon of the gag gene.

Early interest in the region of the HIV genome centered on analysis of the polyprotein fusion product coded therefrom, as well as the construction of a screening assay for AIDS antiviral compounds. To synthesize large quantities of substrate, recombinant gag precursor was expressed by applicants in yeast without the production of the HIV protease.

Applicants are discoverers of the use of gag precursor protein determinants as useful immunogens. This discovery contradicts the commonly-held presumption that surface regions of a pathogen such as a retrovirus are the only sites effective for immunizing the host against a pathogenic microorganism or virus. The HIV gag precursor contains internal components of the virus and they are not accessible to antibody unlike, for example, the envelope protein on the surface of HIV. It was neither anticipated nor expected that gag precursor determinants by themselves are useful as a vaccine against AIDS. In fact, applicants originally isolated the gag precursor of the present invention for the entirely different purpose of preparing a substrate for a proteolytic processing assay.

A principle objective of the present invention is the use of recombinant gag precursor protein as an AIDS vaccine. Methods are disclosed for preparing a novel, water-soluble form of the recombinant protein in large quantities. The product of these methods is also useful as a screening agent in assays for finding antiviral agents.

Purification of recombinant proteins not infrequently requires new methods or novel combinations of old methods, since the composition of the source for recombinant forms, e.g. yeast, are different from conventional sources, e.g., serum. One cannot predict what methods are useful for isolating proteins from recombinant cell cultures on the basis of the knowledge and know-how of methods useful for isolating proteins from classical or otherwise conventional sources. Purification processes designed for preparing vaccines require unusual purity in the product, another indication of the unpredictability in the art. For a similar view, see U.S. Patent 4,624,918.

## BRIEF DESCRIPTION OF THE INVENTION

Applicants disclose the protein yeast recombinant gag precursor of greater than 95% purity or equally pure variant thereof. Experiments by applicants demonstrate that this protein is myristoylated and therefore different in at least one respect from E. coli-produced recombinant gag precursor. Recombinant gag precursor is alternatively expressed in a recombinant insect expression system. The purified gag precursor is useful as a vaccine and in commercial as says of AIDS viral protease.

Applicants also disclose a process of substantially purifying the protein yeast recombinant gag precursor or variant thereof, comprising the steps of

(a) providing a quantity of clarified yeast extract, said yeast extract containing yeast recombinant gag precursor, or variant thereof, expressed in a recombinant yeast expression system;

(b) precipitating the gag precursor by freezing for a period of at least about 24 hours;

(c) pelleting by centrifugation the gag precursor precipitate formed, and thereafter separating the pellet from the supernatant;

(d) mixing the pellet with a solution effective for solubilizing membranes and undesired membrane bound proteins;

(e) repelleting the gag precursor by centrifugation to form a second pellet, and thereafter separating the second pellet from the supernatant;

(f) dissolving the pellet of the preceeding step in a solution effective for solubilizing the gag precursor, to yield denatured pellet; and

(g) subjecting the denatured pellet to hydrophobic interaction chromatography, and isolating fractions with gag precursor therein, yielding thereby substantially purified yeast recombinant gag precursor, or purified variant thereof. The gag precursor then is rendered water-soluble by reverse phase-high pressure liquid chromatography (RP-HPLC) or gel filtration chromatography in an organic phase. The purified gag precursor is useful as a vaccine and in commercial assays of AIDS viral protease.

## BRIEF DESCRIPTION OF THE DRAWINGS

FIG 1: Purification Scheme for recombinant gag precursor with reverse phase high pressure liquid chromatography.

FIG 2: Purification Scheme for recombinant gag precursor with gel filtration chromatography in the organic phase

## ABBREVIATIONS AND DEFINITIONS

AIDS Acquired immunodeficiency syndrome
ARC AIDS-related complex
bp base pairs
gag p55 The gag precursor of HIV, serotype New York #5, with a mobility on gels of about 55 KDa, also known as p55 gag.
gag precursor Any protein product of the gag gene of HIV, or variants thereof. As defined, gag precursor never contains the amino acid sequence that acts as a viral protease.
HIV Generic term for the presumed etiological agent of AIDS, also referred to as strains HTLV-III, LAV, and ARV.
kDa Kilodaltons
kb Kilobases
ORF Open reading frame
Recombinant protein: A polypeptide or oligopeptide expressed by foreign DNA in a recombinant eukaryotic or procaryotic expression system, e.g.,yeast recombinant gag precursor of HIV.
Recombinant eukaryotic expression system: A eukaryotic cell containing a foreign DNA expressing a foreign protein or a foreign oligopeptide, e.g., U9 #5-1 in this application.

## DETAILED DESCRIPTION OF THE INVENTION

### Gag Precursor, p55, and its Variants

The specific protein used for illustrative purposes in the present invention is the recombinant gag precursor of HIV, serotype New York #5, expressed in a recombinant yeast expression system. By convention, proteins are also designated by apparent mobility on denaturing gels. In this case, the particular recombinant gag precursor migrates at a position approximating 55 kDa, so its alternative designation is p55, wherein lower case "p" indicates protein.

It will be apparent to a skilled artisan that the teachings of this invention are readily adaptable to any gag precursor of any strain of HIV provided that the gag precursor does not contain viral amino acid sequences that proteolytically cleave itself.

By way of illustration, the HIV clone of the present invention was cleaved at an introduced SmaI site immediately downstream of the gag translation stop codon, so that the HIV amino acid sequences having proteolytic activity never appear in any gag precursor expression system. By the same principle, the location of the viral protease sequences having proteolytic activity can be ascertained by analysis of the nucleotide sequence of any HIV strain. The DNA clone can then be cut to effectively eliminate any interfering proteolytic sequences after its translation in a recombinant expression system. The gag precursor of the present invention does not derive from the pol or env regions, except for the region of overlap between gag and pol (about 230 bp).

It will be understood that the novel processes and products of the present invention encompass a range of expressed recombinant gag precursors. The processes and products of the present invention also are designed to provide rapid and efficient methods of preparing vaccines containing variants of gag precursor. For example, variations by conservative substitution in the amino acid sequence [defined as sets in Table 1 of Taylor, W.R. J. Mol. Biol. 188, 233 (1986)] generally will not result in any substantial or novel modification of the principles and practice of the present invention. Alternatively, deletions within the coding regions will not in general require any modification(s) of the processes for preparing vaccines discussed herein. Still other variations occur through post-translational modifications, and include proteolytic processing, adenyiation, carboxylation, glycosylation, hydroxylation, methylation, phosphorylation or myristoylation. It will be understood that gag precursor or variant thereof in this application includes any such variations or portions of the amino acid sequence, whether by conservative amino acid substitution, deletion, post-translational processing, ribosome frame shifting or other process, provided that the resulting gag precursor or variant thereof, is immunochemically reactive with antibodies specific for gag p55 of HIV, serotype New York #5.

## Eukaryotic Expression Systems

It is now a relatively straightforward technology to prepare eukaryotic cells expressing a foreign gene. Such eukaryotic cells act as hosts and include yeasts, fungi, plant cells and animal cells. Expression vectors for many of these host cells have been isolated and characterized, and are used as starting materials in the construction, through conventional recombinant DNA techniques, of vectors having a foreign DNA insert of interest. Any DNA is foreign if it does not naturally derive from the host cells used to express the DNA insert. The foreign DNA insert may be expressed on extrachromosomal plasmids or after integration in whole or in part in the host cell chromosome(s), or may actually exist in the host cell as a combination of more than one molecular form. The choice of host cell and expression vector for the expression of a desired foreign DNA largely depends on availability of the host cell and how fastidious it is, whether the host cell will support the replication of the expression vector, and other factors readily appreciated by those of ordinary skill in the art, such as the splicing of secretory sequences onto the structural gene insert for the purposes of gaining simpler modes of purification of the desired recombinant protein, or the insertion in the expression vector of transactivating sequences.

The foreign DNA insert of interest comprises any DNA sequence coding for gag precursor or variants thereof, including any synthetic sequence with this coding capacity or any such cloned sequence or combination thereof. For example, gag precursor protein coded and expressed by an entirely synthetic DNA sequence is encompassed by this invention. Gag precursor DNA is now available, e.g., ATCC Accession No. CRL 8543.

## Expression Vectors

Vectors useful for constructing eukaryotic expression systems for the production of recombinant gag

precursor comprise the DNA sequence for gag precursor or variant thereof, operatively linked thereto with appropriate transcriptional activation DNA sequences, such as a promoter and/or operator. Other typical features may include appropriate ribosome binding sites, termination codons, enhancers, terminators, or replicon elements. These additional features can be inserted into the vector at the appropriate site or sites by conventional splicing techniques such as restriction endonuclease digestion and ligation.

The recombinant yeast expression system exemplified herein was chosen by applicants for the sake of convenience. Yeast vector pKH12-2 was constructed by inserting a 1.5 kb BamHI-SmaI gag fragment of DNA into the yeast expression vector pC1/1-pGAP(B)tADH1. The SmaI site was introduced by site-directed mutagenesis at-a location 5bp downstream and 3′ of the termination codon of the region. Hence, the typical recombinant protein expressed in the yeast cell with this specific construction is full-length gag precursor that is unprocessed due to the absence of HIV proteases in the yeast cell. Proteolytic processing may occur by the presence of endogenous yeast proteases, and it will be apparent that this invention encompasses the resulting fragments generated thereto, provided that they still are large enough to immunochemically react with antibodies specific for a native or natural form of gag precursor.

Yeast expression systems, which are one variety of recombinant eukaryotic expression systems, generally employ Saccharomyces cerevisiae as the species of choice for expressing recombinant proteins. However, it will be apparent to those skilled in the art that, for the expression of gag precursor, the selection of a host strain or cell extends to species from other genera of yeast within the Families Saccharomycetaceae and Cryptococcaceae, including, but not limited to Pichia, Candida, Hansenula, Torulopsis, Kluyveromyces, and Saccharomycopsis. Similarly, selection of a host strain or cell extends to other species of the genus Saccharomyces. Many of the species of this genus are capable of interbreeding with S. cerevisiae and are likely to possess promoters which are analogous or identical to promoters in S. cerevisiae, including but not limited to GAP491, GAL10, ADH2, and/or alpha mating factor promoters. Therefore, it will be apparent to those skilled in the art that, for the expression of recombinant gag precursor polypeptides, the selection of a host strain extends to other species of the genus Saccharomyces, including but not limited to carlsbergensis, uvarum, rouxii, montanus, kluyveri, elongisporus, norbensis, oviformis, and diastaticus.

Yeast vectors useful for constructing recombinant yeast expression systems for expressing gag precursor include, but are not limited to, shuttle vectors, cosmid plasmids, chimeric plasmids, and those having sequences derived from 2-micron circle plasmids. A variety of promoters and other yeast transcriptional control sequences may be used to express the inserted DNA sequence coding for gag precursor, including those of GAP491, GAL10, ADHI or ADHII, PHO5 and the alpha mating factor. The following list of yeast vectors is intended for illustrative purposes only:

pAAH5
pAH5
pAAR6
pFRPn
pGAL10-MFα1
pJC197
pMA56
pABADE8
pAXα11
pAH9
pAH10
pAH21
pMAC561
pLG669
pMA91
pMA301
pYEHBs
pAM82.

Insertion of the appropriate DNA sequence coding for gag precursor, or variant thereof, into these vectors will, in principle, result in a useful recombinant yeast expression system for gag precursor where the modified vector is inserted into the appropriate host cell, by transformation or other means.

Recombinant mammalian expression systems are another means of producing the recombinant gag precursor of the present invention. In general, a host mammalian cell can be any cell that has been efficiently cloned in cell culture. An expression vector for mammalian cells should have a selectable marker, such as the neo gene which confers resistance to certain antibiotics, so that the presence of the vector in

the cell can be detected. DNA-mediated transfection, transvection or transformation of the cell can be accomplished by, e.g., the calcium phosphate technique, electroporation, or microinjection. Transactivating sequences may be necessary for substantial expression, and their insertion into appropriate vectors is a familiar modification of the present invention.

Host mammalian cells useful for the purposes of constructing a recombinant mammalian expression system include, but are not limited to, Vero cells, NIH3T3, GH3, COS, murine C127 or mouse L cells. Mammalian expression vectors can be based on virus vectors, plasmid vectors which may have SV40, BPV or other viral replicons, or vectors without a replicon for animal cells. Detailed discussions on mammalian expression vectors can be found in Gluzman, Y. (ed.) "Eukaryotic Viral Vectors" Cold Spring Harbor Laboratory 1982; Pouwels, P.H. et al. "Cloning Vectors: A Laboratory Manual" Elsevier 1985; Rigby, P.W.J., J. Gen. Virol. 64, 255 (1983); Subramani, S. et al. Anal. Biochem. 135, 1 (1983); and Glover, D.M. (ed.) "DNA Cloning: A Practical Approach," IRL 1985, Vols. I and II.

## PURIFICATION OF GAG PRECURSOR

To counteract instability and breakdown of gag precursor during purification procedures, buffers generally incorporate protease inhibitors such as phenylmethylsulfonyl fluoride (PMSF) or EDTA. The preferred inhibitors are EDTA in combination with benzamidine, pepstatin, aprotinin, PMSF and E-64.

Typical protease inhibitors suitable for the procedures and protocols of this invention include but are not limited to metal-chelating agents, heavy metal ions, SH blocking reagents, substrate-like compounds of proteases or polypeptides that inhibit proteases. Some useful protease inhibitors are provided as follows:

$Ag^{++}$, $Hg^{++}$, $Cu^{++}$ and other heavy metal ions
antithrombin III
antithrombin III - heparin
$\alpha_1$-antitrypsin
aprotinin
basic amino acids
benzamidine
bestatin
$\alpha,\alpha'$-bipyridyl, Na-fluoride
4-bromo-phenacyl bromide
chicken egg white trypsin inhibitor
chymostatin
citrate
cysteine
4-dinitrophenol diethylphosphate
DFP (diisopropylphosphofluoridate)
DTT (dithiothreitol)
E-64 [trans-epoxysuccinyl-L-leucylamido-(4 guanidine)-butane, Boehringer Mannheim]
EDTA and other chelating agents
formaldehyde
guanidinium chloride
heparin
hirudin
4-hydroxymercuribenzoate
iodoacetamide
iodoacetic acid
leupeptin
$\alpha_2$-macroglobulin
mercaptoethanol
p-mercuribenzoate
mercuric chloride
$\alpha$-microglobulin
$\alpha$-N-(p-nitrobenzyl-oxycarbonyl)-L-arginylchloromethyl ketone
oxalate
pepstatin from a variety of sources, including Streptomyces

1,10-phenanthroline
2-phenanthroline
phenothiazine-N-carbonyl chloride
phosphoramidone
PMSF (phenylmethylsulfonyl fluoride)
pyrophosphate
SH-blocking reagents
silver nitrate
soybean trypsin inhibitor
p-toluene sulfonyl fluoride
TLCK(L-1-chloro-3-(4-tosylamido)-7-amino-2-heptanone-hydrochloride)
TRITON X-100 and other detergents at low concentrations
TPCK(L-1-chloro-3-(4-tosylamido)-4-phenyl-2-butanone)
trypsin inhibitor from hen egg
ZPCK (benzyloxycarbonyl-L-phenylalanine)

Buffered solutions containing one or more of the listed inhibitors may be adapted to one or more steps in the process of purifying gag precursor. A preferred buffer suitable for purification of the gag precursor contains EDTA, benzamidine, pepstatin A, aprotinin, PMSF and E-64.

## YEAST CLARIFIED EXTRACT

Yeast cells transformed with expression vectors coding for gag precursor, p55, or variants thereof, are grown and harvested. If storage of the cells is desired, the cells are washed in buffer, e.g. PBS [phosphate buffered saline consisting essentially of 1mM $CaCl_2$ (anhydrous), 2.7 mM KCl, 1.47 mM $kH_2PO_4$, 0.5 mM $MgCl_2 \bullet 6H_2O$, 137 mM NaCl, 8.06 mM $Na_2HPO_4 \bullet 7H_2O$ in water], to form a cell paste. Storage of the cell paste is typically done by freezing in liquid $N_2$.

Purification typically begins as follows. A batch of frozen cell paste is thawed and suspended in buffer containing proteolytic inhibitors, e.g. PBS in 2mM PMSF, hypertonic phosphate buffers, preferably reagent 3 [0.1M HEPES pH 7.5, 10 mM EDTA, 10mM benzamidine, 1.0 µg/ml pepstatin A, 0.13 Trypsin Inhibitory Units (TIU)/ml aprotinin, 2mM PMSF, 0.1mM trans-epoxysuccinyl-L-leucylamido-(4 guanidino)-butane]. Low temperature conditions, e.g. 4° C, are also preferred in the interest of lowering to a minimum any residual endogenous proteolytic activity. Cells then are disrupted, preferably by mechanical means. A variety of commercially available cell disrupters and fluidizers are suitable and easily adaptable.

Yeast cell disruption results in a crude extract. It is necessary at this point to clarify the extract by removing cell debris so that mechanical clogging in subsequent purification steps is avoided. Centrifugation at about 14,000 x g for 45 minutes at 4° C has been found adequate, but it is readily understood that different centrifugation speeds for different times at different temperatures are also possible and easily rendered operable. The presence of gag precursor can be ascertained by denaturing gels combined with immunoblotting.

Further steps in the purification process are performed on the 14,000 x g supernatant, also designated the yeast clarified extract. The pellet is discarded, except to the extent that samples are retained for analytical determinations. The yeast clarified extract is routinely frozen at -70° C. Applicants have found that overall yield improves if the clarified extract is stored at -70° C for long periods of time, e.g. at least 24 hours, preferably at least 72 hours.

## DENATURED PELLET

Applicants have also discovered that freezing the yeast clarified extract for at least 24 hours results in spontaneously precipitated gag precursor, so that simple centrifugation or pelleting of the thawed extract results in substantial purification. After thawing of the frozen yeast clarified extract, successive steps of pelleting followed by homogenization of the pellet in various buffers with or without detergents are performed to wash the precipitated gag precursor so as to remove membranes, undesired membrane bound proteins and other membraneous material. The resulting washed pellet then is solubilized to make a

denatured pellet.

At the least, preparation of the denatured pellet as outlined in the previous paragraph encompasses the following steps:

(a) thawing the frozen yeast clarified extract after at least 24 hours of freezing;

(b) pelleting the gag precursor by centrifugation at between about 10,000xg and 100,000xg for a period of between about 10 min and about 120 min, and thereafter separating the supernatant from the pellet; then

(c) mixing and homogenizing the pellet with a buffered solution effective for solubilizing membranes and membrane-bound proteins. The solution typically comprises buffer with a detergent.

(d) Repelleting the gag precursor follows, and it is performed by centrifugation at between 10,000xg and 100,000xg for a period of between about 10 min and about 120 min, and thereafter separating the supernatant from the pellet; then

(e) the washing steps of (c) and (d) supra are repeated 0-3 times with the same solution, or a different buffered detergent, or buffer alone, or any combination thereof. The product is a washed pellet.

(f) The washed pellet is dissolved in buffered denaturant or chaotropic agent, yielding a denatured pellet ready for hydrophobic interaction chromatography.

More specifically, thawing as a first step [step (a) supra] generally is performed at room temperature or at 37° C, but other temperatures in the range of 4° C - 40° C are feasible and within the scope of the present invention.

Centrifugation forces for pelleting gag precursor according to steps (b), (d) or (e) can be varied, as well as the spinning time and temperature. It is readily understood that modifications in such variables in the pelleting process are easily rendered operable, usually by visual inspection.

In step (c) supra, the detergent in the buffered solution for solubilizing membranes and membrane-bound proteins is any suitable non-ionic or ionic detergent, or any suitable combination thereof, including but not limited to detergents of the nonoxynol series, octoxynol series, polyoxyethylene alcohol series, polyoxyethylene (20) sorbitan mono-oleate series, deoxycholate, or octylglucopyranoside, and the like. Zwitterionic detergents are also useful and suitable agents. Preferably, enough buffered solution with detergent should be added to the pellet of step (c) to yield a final concentration of between about 1% (w/v) and about 10% (w/v) octylphenoxy polyethoxyethanol (marketed under the trademark TRITON X-100). Most preferably, the final concentration is about 5% (w/v) TRITON X-100, in Reagent 3.

Once the washed pellet of steps (d) or (e) is prepared, it is denatured in step (f) with a denaturant or chactropic agent to render it adaptable for the next step of hydrophobic interaction chromatography. Generally, any denaturant of formula I is suitable and feasible for the purposes of denaturation:

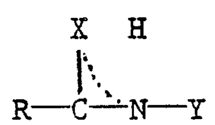

I

wherein

R is amino, loweralkylthio, loweralkyloxy, or sulfur;

X is amino, sulfur or oxygen; and

Y is hydrogen or amino.

This formula includes guanidine•HCl, wherein R is amino, X is amino and Y is hydrogen. The preferred agent for denaturing the washed pellet in step (f) is guanidine•HCl at concentrations between about 2M and about 8M in Reagent 3, most preferably 6M. Subsequent dilution of the denaturant may be performed immediately before hydrophobic interaction chromatography.

## HYDROPHOBIC INTERACTION CHROMATOGRAPHY

The gag precursor in the denatured pellet is now further purified by hydrophobic interaction in column chromatography. The preferred resin for this purpose is PHENYL-SEPHAROSE (Pharmacia). Other resins are also suitable, and include, for example, OCTYL-SEPHAROSE (Pharmacia), BUTYL-SEPHAROSE (Pharmacia) and similar derivatives of agarose, such as the ω-amino alkyl agaroses of Miles Research or

hexyl-agarose. Denaturants of formula I are used, preferably guanidine•HCl.

In a typical protocol, the column is equilibrated in buffer, or preferably buffered 2M guanidine•HCl, then denatured pellet is loaded, the column is washed in buffer until elution reaches a baseline in absorbance. The column, now containing gag precursor bound thereto, is developed with a gradient of buffer in increasing concentration of denaturant(s). In effect, as the concentration increases, selective solubilization occurs. Fractions coming off the column are collected and monitored for the presence of gag precursor, by e.g. SDS-polyacrylamide gel electrophoresis or Western blotting. Pooled fractions contain gag precursor. On Phenyl-SEPHAROSE, yeast recombinant gag precursor elutes at about 3M guanidine•HCl.

## WATER-SOLUBLE RECOMBINANT YEAST GAG PRECURSOR

Another discovery of applicants is that processing the protein at this stage through reverse phase high pressure liquid chromatography (RP-HPLC) under certain conditions results in a water-soluble gag precursor product. In the alternative, applicants have also discovered that a substitute protocol of gel filtration under certain conditions results in a water-soluble gag precursor product.

### A. RP-HPLC

In general, any reverse phase high pressure liquid chromatography method using gradient elution can be employed, provided that the column is packed with suitably porous particles coated with alkyl or phenyl groups which act as a non-polar stationary phase. $C_4$, $C_8$, $C_{18}$ or phenyl groups are suitable for the process of the present invention. $C_{18}$ is the preferred group.

The column is equilibrated in a stationary phase of about 0.1% (v/v) trifluoroacetic acid (TFA) in 20% (v/v) acetonitrile in water, typical conditions for RP-HPLC. After the sample of pooled fractions is concentrated and applied, the column is developed with a mobile phase of the same constitution as the stationary phase but with a gradient of increasing acetonitrile concentration. Fractions identified to contain gag precursor are pooled, then dried in vacuo and resuspended in sterile distilled water. The resulting protein is gag precursor (≥95%), which is soluble in water or 1M urea.

Other suitable stationary phase-mobile phase combinations include but are not limited to phosphoric acid with acetonitrile gradients of 10-40%; phosphoric acid or TFA with isopropanol gradients. Other modifications of HPLC useful for preparing water-soluble gag precursor are readily appreciated by those skilled in the art, and include the following: use of other columns, mobile phases, organic phases; determination of water content in the mobile phase, ionic strength, pH, temperature; and evaluation of ion-pair and ion-exchange effects. See, for example, Waterfield, M.D., "Separation of Mixtures of Proteins and Peptides by HPLC, "in Darbre, A. (ed.) Practical Protein Chemistry Wiley 1986, pp. 181-205.

### B. GEL FILTRATION IN ORGANIC SOLVENTS

Water-soluble gag precursor also can be produced by gel filtration chromatography in organic solvents instead of RP-HPLC. The gel filtration matrix must be compatible with the organic solvents employed. Some of the suitable matrixes include SEPHAROSE (Pharmacia), SEPHAROSE CL (Pharmacia), SEPHACRYL based resins (Pharmacia), SEPHADEX LH (Pharmacia), SEPHASORB (Pharmacia) and BIO-BEADS S-X (Bio-Rad). The preferred matrices are SEPHADEX LH or any SEPHACRYL based resin, including SEPHACRYL S-300 or SEPHACRYL S-200HR, of which the latter has the most preferred characteristic of satisfactorily separating protein from guanidine•HCl salt contaminants.

In principle any organic solvent may be employed. Typically, the solvent includes low concentrations of an aqueous acid, such as TEA or phosphoric acid, in organic solvent(s) such as acetonitrile, isopropanol, methanol or methylene chloride. A preferred range of solvent systems is 0.1% (v/v) TFA/≧30% acetonitrile/$H_2O$. The most preferred solvent system is approximately 0.1% (v/v) TEA/70% acetonitrile/$H_2O$.

## ADDITIONAL STEPS

Other conventional or known steps normally used in purification of recombinant proteins may be added to the process of purifying gag precursor. These steps include, but are not limited to:

(a) selective adsorption or partition on a solid-phase, e.g. silica gel, calcium phosphate charcoal, or celite alumina;

(b) selective extraction with solvents or reagents. Extraction with other solvents or reagents for other purposes may be needed in different steps.

(c) Precipitation is another step useful for isolating gag precursor, and can be performed with salts such as ammonium sulfate, or on a pH gradient by isoelectric precipitation. Other methods include.

(d) chromatography by any standard method, including paper, thin-layer, molecular sieve, molecular exclusion, ion-exchange, ligand affinity, immunoaffinity, or by electrophoresis;

(e) high pressure liquid chromatography, be it by size exclusion, adsorption or partition in the normal phase;

(f) solvent fractionation by two phase extractions, e.g., with PEG and dextran [Anderson, E. et al., Ann. N.Y. Acad. Sci. 413, 115 (1983)],

(g) dialysis, ultrafiltration, concentration or diafiltration;

(h) density-gradient centrifugation;

(i) electrofocusing;

(j) freeze drying, lyophilization; or

(k) crystallization.

This list is by no means exhaustive. Its order is not an indicaton of the preferred order of purification. It will be understood that a successful purification of gag precursor may be conducted with any or all of steps (a)-(k) in conjunction with precipitation by freezing, hydrophobic interaction chromatography, and water solubilization by either RP-HPLC or gel filtration chromatography in organic phases. Steps (a)-(k), as well as other purification techniques, may be inserted as an additional step or steps at any point or points of the purification process.

The vaccines of this invention may be effectively administered, whether at periods of pre-exposure and/or post-exposure, in combination with effective amounts of the AIDS antivirals, immuno modulators, antiobiotics, or vaccines of Table I [source: Market Letter, Nov. 30, 1987, p. 26-27; Genetic Engineering News, Jan. 1988, Vol. 8, p. 23.]

TABLE I[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR (zidovudine; AZT) | Burroughs Wellcome | AIDS, advanced ARC<br><br>pediatric AIDS, KS, asympt HIV, less severe HIV, neurological involvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with RETROVIR |
| ZOVIRAX (acyclovir) | Burroughs Wellcome | AIDS, ARC, in comb with RETROVIR |

B. Immunomodulators

| | | |
|---|---|---|
| ABPP (bropirimine) | Upjohn | Advanced AIDS, KS |
| AMPLIGEN (mismatched RNA) | DuPont HEM Research | ARC, PGL |
| (Anti-human alpha interferon antibody) | Advanced Biotherapy Concepts | AIDS, ARC, KS |

| Drug Name | Manufacturer | Indication |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| Drug Name | Manufacturer | Indication |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & RETROVIR therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the vaccines of this invention in the treatment or prevention of AIDS and diseases of similar character caused by HIV.

It will be understood that the scope of combinations of the vaccines of this invention with AIDS antivirals, immunomodulators, antibiotics or vaccines is not limited to the list in the above Table, but includes in principle any combination with any pharmaceutical composition useful for the treatment of AIDS. The AIDS or HIV vaccines of this invention include vaccines to be used pre- or post-exposure to prevent or treat HIV infection or disease, and are capable of producing an immune response specific for the immunogen.

The form of the immunogen within the vaccine takes various molecular configurations, and a carrier, vector or adjuvant may be added as an immunological vehicle according to conventional immunological testing and practice. For example, one embodiment of the present invention is the prophylactic vaccination

of patients with a suspension of alum adjuvant as vehicle and recombinant yeast gag precursor (or variant thereof) as immunogen.

It will be apparent to those skilled in the art that the immunogen may alternatively consist of gag precursor amino acid sequences (or variant thereof) bound, either covalently or non-covalently, to determinants known to enhance immunogenicity. Such determinants are typically amino acid sequences that induce helper T-cell, cytolytic T-cell or B-cell immunity. It is desirable to delete amino acid sequences, if any, that induce suppressor T-cell immunity. One example of these determinants is the Hepatitis B preS2 amino acid sequence which is known to enhance helper T-cell and B-cell immunity.

Once it is determined by conventional immunological practice that a determinant for enhancing immunogenicity is desirable, it may be fused covalently to the antigen (Ag), e.g., the carboxy terminus of the entire Hepatitis B core antigen or portion thereof fused via a peptide bond or other covalent linkage to the amino terminus of recombinant p55, or recombinant yeast Ty:Ag particles. These fusion constructions are readily prepared, for example, by splicing DNA coding for the corresponding structural proteins, expressing the spliced DNA, and isolating the fusion protein product. Alternatively or additionally, non-covalent interactions that bind antigen to such determinants may produce particulate forms of various sizes or simple aggregates, any of which are useful in the enhancement of immunogenicity. Mention should be made of other vaccine forms: the peptide cocktail containing a wide spectrum of MHC restricted peptides, and DNA recombinants of DNA sequences for Ag and vaccinia vector.

Adjuvants may or may not be added during the preparation of the vaccines of this invention. Alum is the typical and preferred adjuvant in human vaccines. Others include incomplete Freund's adjuvant or liposomes.

The preparation, formulation, and testing of these vaccines with enhanced capacity to generate specific immunity, be it by using determinants that induce T-cell immunity, cytolytic T-cell or B-cell immunity, require generally conventional and routine skills in the immunology, recombinant DNA and related arts.

In the examples the following materials were purchased from commercial sources: guanidine•HCl, International Biotechnologies, Inc.; TRITON X-100, Boehringer Mannheim.

Throughout this application, the gag precursor or HIV p55 can be monitored by denaturing gels such as electrophoresis on SDS polyacrylamide gels, by Western blotting [Burnette, W.N. Anal. Biochem. 112, 192 (1981), or both.

## EXAMPLE 1

### Construction of Yeast Expression Vector for HIV Gag Precursor with an Introduced Stop Codon Immediately Downstream of the Gag Precursor Stop Codon

Plasmid pBL4-3-2 (alternatively pNL4-3) is pUC18 with a ca. 22 kb insert comprising 9.7 kb of HIV DNA, consisting of the 5′ half of New York HIV isolate #5 fused to the 3′ half of the LAV isolate [A. Adachi et al., J. Virol. 59, 284-291 (1986)]. A variety of other plasmids containing AIDS viral sequences can substitute for pBL4-3-2 (or pNL4-3) in this procedure for the construction of yeast expression vector for HIV gag precursor and the choice of the appropriate genetic engineering manipulations on any such plasmid is within the skill of a molecular biologist.

Plasmid pBL4-3-2 (alternatively pNL4-3) was digested with HindIII to yield a partial digest and the resulting 5.7 kb HindIII fragment bearing the gag-pol coding regions was subcloned into pUC13 to yield two identical plasmids, designated P1 and P2. Both plasmids were then handled separately in an identical fashion as described below. Plasmid P1 (or P2) was then digested with HgaI plus NdeI. The resulting 4.4 kb HgaI-NdeI gag-pol fragment was made blunt-ended with the Klenow fragment of DNA polymerase I. (The filled-in HgaI site generates a blunt-end at position +3 of the gag coding sequence.) The 4.4 kb blunt-ended fragment was ligated with a synthetic oligonucleotide adapter having the structure:

```
5'-AGCTTGGATCCACAAAACAAAAT-3'
3'-     ACCTAGGTGTTTTGTTTTA-5'
```

Ligation of the adapter to the flush-ended HgaI site rebuilds the ATG initiation codon for the gene and

adds a yeast-like nontranslated mRNA leader plus HindIII and BamHI cloning sites. Ligation of this adapter to the flush-ended NdeI site results in the addition of a translation stop codon plus BamHI and HindIII cloning sites 27 bp downstream of the natural pol translation stop codon. The ligation mixture was then digested with HindIII and the 4.4 kb HindIII fragment bearing the gag-pol coding regions was gel-purified and then cloned into the HindIII site of pAAH5 [G. Ammerer Methods in Enzymology 101, 192-201 (1983)]. Following transformation into E. coli, plasmid p64-30 was obtained from the original plasmid P1 and p50-81 was derived from plasmid P2.

The plasmid p64-30 was digested with BamHI plus HincII and the 1760 bp BamHI-HincII fragment (bearing the gag coding region) was gel-purified and then subcloned into M13mp18 which had been previously digested with BamHI plus HincII. Using the technique of oligonucleotide-directed mutagenesis [M.J. Zoller et al., Methods in Enzymology 100, 468-500 (1983)], a SmaI site was introduced at 5 bp downstream of the translation stop codon. The oligonucleotide used in the mutagenesis had the following structure:

5′-CCCCCCGGGCTTTATTGTGAC-3′

The mutagenized plasmid was digested with BglII plus HincII and the 0.44 kb BglII-HincII fragment containing the 3′-end of the gag coding region was gel-purified. The plasmid p50-81 was digested with BamHI plus BglII and the 1.3 kb BamHI-BglII fragment containing the 5′-end of the gag coding region was gel-purified. The aforementioned 0.44 kb and 1.3 kb fragments were ligated together with the vector pGEM-4 which had been previously digested with BamHI plus HincII. The resulting plasmid, pKH-GAG (4.6 kb), contains a reconstructed gene with a yeast-like 5′ non-translated leader sequence and a SmaI site 5 bp downstream of the translation stop codon.

pKH-GAG was digested with BamHI plus SmaI. The resulting 1.5 kb BamHI-SmaI gag fragment was flush-ended with the Klenow fragment of DNA polymerase I and gel-purified. The yeast expression vector pC1/1-pGAP(B)tADH1 contains the constitutive promoter for the yeast glyceraldehyde-3-phosphate dehydrogenase gene (GAP491) plus the transcriptional terminator for the yeast ADHI gene. A unique BamHI site is located between the promoter and terminator elements. This vector is functionally identical to the GAP491 expression vector previously described by P. Kniskern et al., Gene 46, 135-141 (1986). pC1/1-pGAP(B)tADH1 was digested with BamHI and made flush-ended with the Klenow fragment of DNA polymerase I. The vector fragment and the 1.5 kb gag fragment were ligated together to yield pKH12-2. This recombinant plasmid was then transformed into the Saccharomyces cerevisiae strain U9 (MATa, leu2-04, ade1, ura3, cir°). Strain U9 is a spontaneous ura3 isolate derived from the S. cerevisiae strain 2150-2-3 (MATa, leu2-04, ade1, cir°).

Transformant clones were selected and amplified. Cell lysates were prepared, electrophoresed in polyacrylamide gels and Western blotted to nitrocellulose. Immunoblotting with HIV-positive human sera demonstrated the presence of an HIV-specific 55 kilodalton protein in the transformants. This 55-kDa protein also reacted with anti-p24 antibodies by RIA, confirming its identity as the HIV gag p55 protein.

The clonal isolate U9#5-1 was selected to prepare a Master Seed (PF413) for all further fermentation and protein purification studies. The master seed was stored frozen at -60 to -80° C in the presence of 17% (v/v) glycerol.

Samples of U9#5-1, P1 and P2 were deposited on 25 April 1988 at the American Type Culture Collection, 12301 Parklawn Drive, Rockville, MD 20852, with Accession Nos. 20876, 67669, and 67670, respectively.

## EXAMPLE 2

### Preparation of Yeast Clarified Extracts

Frozen recombinant yeast cells of Example 1 were thawed. Breaking buffer [0.1M HEPES pH 7.5, 10mM EDTA, 10mM benzamidine, 1µg/ml pepstatin A, 0.13 TIU/ml aprotinin, 2mM PMSF, 0.1mM transepoxysuccinyl-2-leucylamido-(4-guanidino)-butane was added to make an approximately 25% (w/v) cell suspension. Cells were broken by pressure induced lysis at 4° C. After centrifugation at 14,000xg for 45 min at 4° C, the pellet was discarded. The supernatant was the yeast clarified extract and was frozen routinely at -70° C for at least 72 hrs.

## EXAMPLE 3

### Purification of Yeast Clarified Extract, First Variation

#### A. Denatured Pellet

An 800 ml aliquot of frozen yeast clarified extract of Example 2 was thawed in a room temperature water bath, then centrifuged at 30,000 rpm (105,000xg) for 60 min at 4° C. The supernatant was discarded, and a pellet in each centrifuge tube retained. Quantities of 5 ml of 5% (w/v) TRITON X-100 (Boehringer Mannheim) in Reagent 3 [0.1M HEPES (pH 7.5), 10mM EDTA, 10μg/ml Pepstatin A, 0.13 TIU/ml Aprotinin, 10mM Benzamidine•HCl, ·25-M transepoxysuccinyl-L-leucylamido-(4 guanidino)-butane, 0.1% (w/v)2-mercaptoethanol] were added to the pellet of each centrifuge tube to solubilize the pelleted membranes and associated membrane-bound proteins. The pellets were homogenized, resulting in a suspension in about 2.5% (w/v) TRITON X-100. The suspension was repelleted at 30,000 rpm for 30 min. at 4° C. The resulting Triton Supernatant I was discarded, but the pellet in each centrifuge tube retained. Each pellet then was solubilized again, by adding to each pellet 5 ml of Reagent 4 [6M guanidine•HCl, 0.1M HEPES (pH 7.5), 10mM EDTA, 10μg/ml Pepstatin A, 0.13 TIU/ml Aprotinin, 10 mM Benzamidine•HCl, 25μM trans-epoxysuccinyl-L-leucylamido-(4 guanidino)-butane, and 0.1% (w/v)2-mercaptoethanol], followed by homogenization, and finally mixing overnight on an end-over-end mixer under refrigeration. The resulting mixture was the denatured pellet which may be stored at -20° C, or at -70° C after 15 x dilution in Reagent 4.

#### B. Hydrophobic Interaction Chromatography

A column was prepared of Phenyl-Sepharose (Pharmacia) packed in Reagent 3. After loading the denatured pellet, the column was developed with Reagent 3. The large break through peak of 280nm absorbing material was collected and discarded. The column was subjected to linear gradient elution of Reagent 3 to Reagent 4. In effect, as the guanidine•HCl concentration increases, selective solubilization occurs on the column. Fractions were collected and those containing recombinant p55 gag protein (eluting at about 3M guanidine•HCl) were identified by SDS polyacrylamide gel electrophoresis, yielding the Phenyl-SEPHAROSE pool. Analytical samples were prepared for electrophoresis by MeOH precipitation in the presence of carrier BSA to remove the denaturant guanidine•HCl. Guanidine•HCl concentration was monitored and standardized by electrical conductivity measurements. The Phenyl-SEPHAROSE pool may at this point be dialyzed against 10mM HEPES, which quantitatively precipitates p55 (85-90% pure). The precipitate can be solubilized in a solution of 6M guanidine•HCl and 6% β-mercaptoethanol.

#### C. Water-Soluble Recombinant Yeast gag precursor protein

The Phenyl-SEPHAROSE pool was concentrated, then rendered water soluble by processing through a $C_{18}$ reverse phase HPLC (High Pressure Liquid Chromatography) system. The HPLC column was equilibrated with Mixture 1, containing one volume of Reagent 5[1.0 ml trifluoroacetic acid in 999 ml acetonitrile] and four volumes of Reagent 6[1.0 ml of trifluoroacetic acid in 999 ml water]. The concentrated Phenyl-SEPHAROSE pool was prepared by heating at 95° C for 10 min, then centrifuged for 10 min at 14,000xg to remove residual debris. A sample of the supernatant, containing the gag precursor dissolved in Reagent 4, was injected into the equilibrated HPLC column. The solvent delivery system then washed the column by the successive steps of washing with Mixture 1, eluting with a buffer gradient of Mixture 1 to Mixture 2 [four volumes of Reagent 5 and one volume of Reagent 6], and finally washing with Mixture 3 [nine volumes of Reagent 5 and one volume Regent 6]. The major peak absorbing at 260nm was found to be water-soluble yeast recombinant gag precursor in substantially purified form (≥95%), as judged by silver staining on SDS gels after electrophoresis or Western blotting. Solvent was removed by rotary evaporation, then sterile distilled water was added. This HPLC-purified gag precursor was found to be completely soluble in water or in 1M urea, in contrast with the aggregated material found after cell lysis, which material was only soluble in strong denaturants. Overall yield after HPLC was in the range of 2 to 4mg protein from each 50 ml of a 20% suspension of cells.

Sterile filtration and alum adsorption were routinely carried out to prepare the appropriate vaccine.

## EXAMPLE 4

### Purification of Yeast Clarified Extract, Second Variation

#### A. Denatured Pellet

An 800 ml aliquot of frozen yeast clarified extract of Example 2 was thawed in a room temperature water bath, then diluted to a volume of 1600 ml with Reagent 3 to reduce unwanted precipitation. The resulting mixture was centrifuged at 14,000 rpm (30,000xg) for 30 min at 4° C. The supernatant was frozen at -20° C (Supernatant I), and the pellet in each centrifuge tube retained. A volume of 10 ml of a 5% (w/v) TRITON X-100 (Boehringer Mannheim) in Reagent 3 was added to the pellet of each centrifuge tubes to solubilize the pelleted membranes and associated membrane bound proteins. The pellets were homogenized, resulting in a suspension in about 5% (w/v) TRITON X-100. Then the suspension was frozen at -20° C (Frozen Pellet).

Additional operations on the frozen Supernatant I were found to increase yield by about 50% for large scale operations, as follows. Frozen Supernatants I were thawed in a room temperature water bath, centrifuged at 14,000 rpm (30,000xg) for 30 min at 4° C, and the pellets thereto treated each with another 10 ml volume of 5% (w/v) TRITON X-100 in Reagent 3 and then homogenized, yielding homogenized Residual Pellets.

Frozen pellets were thawed in a room temperature water bath, and combined with Residual Pellets in one container. Then the combined pellet was brought to a volume of about 200 ml with 5% (w/v) TRITON X-100 in Reagent 3, and the mixture was homogenized, then centrifuged at 14,000 rpm for 30 min at 4° C. The resulting Triton Supernatant I was discarded, then 20 ml of 5% (w/v) TRITON X-100 in Reagent 3 added, followed by homogenization. The mixture was brought to a volume of about 200 ml with 5% (w/v) TRITON X-100 in Reagent 3, homogenization performed, and centrifugation repeated at 14,000xg for 30 min at 4° C. The resulting Triton Supernatant II was discarded, the pellet then treated with 25 ml of Reagent 4, homogenized and mixed overnight on an end-over-end mixer under refrigeration. The resulting mixture is the denatured pellet, which may be stored at -20° C, or at -70° C after 15x dilution in Reagent.

#### B. Hydrophobic Interaction Chromatography

A column was prepared of Phenyl-Sepharose packed in Column Buffer [two volumes of Reagent 3 and one volume of Reagent 4]. Thus the column was equilibrated in buffer containing 2M guanidine•HCl instead of just buffer (as in Example 3). Before loading on the column, the denatured pellet was centrifuged at 30,000 rpm (105,000xg) for 30 min at 4° C to remove residual debris, and the supernatant brought to 2M guanidine•HCl by the addition of calculated quantities of Reagent 3. After loading, the column was developed in Column Buffer, a 2M guanidine•HCl buffer. The large breakthrough peak of 260 nm absorbing material was collected and discarded.

The column was then subjected to linear gradient elution of Column Buffer to Reagent 4, i.e. a 2M to 6M guanidine•HCl gradient. In effect, as the guanidine•HCl concentration increases, selective solubilization occurs. Since the gag precursor protein did not elute in 2M guanidine•HCl with the void volume, hydrophobic interaction is the probable explanation for the binding of the protein to Phenyl-SEPHAROSE.

Fractions coming off the column were collected and those containing recombinant gag precursor protein were then identified by SDS polyacrylamide gel electrophoresis, yielding after appropriate pooling the Phenyl-Sepharose pool. Gag precursor protein eluted at about 3M guanidine•HCl. Guanidine•HCl concentration was monitored and standardized by electrical conductivity measurements.

#### C. Water-Soluble Recombinant Yeast p55 gag Precursor Protein

The Phenyl-Sepharose pool was concentrated, then rendered water-soluble by repeating the HPLC

procedure in Example 3C.

EXAMPLE 5

Purification of Yeast Clarified Extract, Third Variation, Gel Chromatography as Final Step.

The Phenyl-Sepharose pool of Example 4B was concentrated, then applied to a column of SEPHACRYL S-200HR equilibrated in 70% $CH_3CN$/0.1% TFA/$H_2O$. The column was developed in the same solvent and fractions collected, dried in vacuo and resuspended in water. Gag precursor containing fractions were identified by electrophoresis on polyacrylamide gels. The resulting gag precursor preparations were ≥95% pure and were indistinguishable from the RP-HPLC purified material with respect to water solubility.

EXAMPLE 6

In Vivo Immunogenicity

A. African green monkeys

Alum-adsorbed recombinant yeast gag precursor, purified according to Example 2, was inoculated into African green monkeys at several dose levels. Each dose was delivered intramuscularly at 0 and 1 months. The dose levels were 0, 1.0, 10.0 and 100.0 mcg; three animals were used for each dose level. The monkeys were bled 2 weeks following the second dose and the development of anti-gag precursor antibody was assessed by specific enzyme-linked immunoadsorbent assay (ELISA). All of the animals inoculated with either two 10.0 mcg or two 100.0 mcg doses developed significant anti-gag precursor antibody levels. Positive levels also were achieved in two of three animals receiving two 1.0 mcg doses. None of the animals inoculated with 0 mcg doses (alum placebo) developed anti-gag precursor titers. See Table II.

TABLE II

| Animal # | Dose level (mcg) | ELISA titers at 2 wks following second dose |
|---|---|---|
| 1 | 100.0 | 1:62,500 |
| 2 | 100.0 | 1:62,500 |
| 3 | 100.0 | 1:62,500 |
| 4 | 10.0 | 1:12,500 |
| 5 | 10.0 | 1:62,500 |
| 6 | 10.0 | 1:12,500 |
| 7 | 1.0 | 1:500 |
| 8 | 1.0 | <1:10 |
| 9 | 1.0 | 1:20 |
| 10 | 0 (alum placebo) | <1:10 |
| 11 | 0 (alum placebo) | <1:10 |

B. Mice

Alum-adsorbed recombinant yeast gag precursor, purified according to Example 2, was inoculated into mice (strain ICR) at several dose levels. Each dose was delivered once intraperitioneally. The dose levels were 0.1, 1.0, 10.0 and 100.0 mcg. Ten mice were used for each dose level. The animals were bled 28 days following inoculation and the development of anti-gag precursor was assessed by specific enzyme-linked immuno-adsorbent assay (ELISA). The number of individual antibody-positive animals at each dose level was used to calculate a 50% effective dose ($ED_{50}$) for the antigen. This was the dose level which was expected to seroconvert, by induction of anti-gag precursor antibody, one-half of the inoculated mice. The $ED_{50}$ for gag precursor was 1.5 mcg.

C. Chimpanzees

Alum-adsorbed recombinant yeast gag precursor, purified according to Example 2, is inoculated intramuscularly into two chimpanzees in 100.0 mcg doses at 0, 1 and 6 months. An additional chimpanzee is inoculated similarly with alum placebo. The animals are assessed for development of anti-gag precursor antibody by a schedule of bleedings and analysis by ELISA. The animals are also assessed throughout the trial for the development of any immunological abnormalities.

At four weeks following the last inoculation, all three chimpanzees are challenged with a titrated dose of intact HIV, comprising 25.0 chimpanzee infectious doses delivered intravenously. Following challenges, the chimpanzees are assessed for persistent HIV infection, as well as specific antibody and any immunological abnormalities.

D. Additional testing on Mice

To show that gag precursor protein primes the immune system for rapid response to HIV envelope protein following challenge of the primed animal with intact HIV, the following experimental plan is followed.

Mice are inoculated intraperitoneally at various days with 10.0 mcg doses of alum-adsorbed recombinant yeast gag precursor, purified according to Example 2. Each mouse is challenged with a single sub-immunogenic dose of intact HIV. Each animal is assessed for the development of antibodies specific for the HIV envelope protein to show whether the mice inoculated with gag precursor develop specific anti-envelope antibodies while the alum placebo mice did not.

## EXAMPLE 7

### Yeast Recombinant Gag Precursor is Myristoylated

Yeast cells expressing gag precursor of Example 1, as well as control non-expressing yeast cells, were labeled with $^{3}H$-myristic acid (New England Nuclear) according to Towler, D. et al. Proc. Nat'l Acad. Sci., 83, 2812-2816 (1986). Labeled cell pellets were lysed in HEPES pH 7.5 (100mM), EDTA (10mM), pepstatin A (10uM), benzamidine (10mM), aprotinin (0.13 TIU/ml), E-64 (25 uM), NaCl (150mM), NP-40 (1%) Nadeoxycholate (1%) and SDS (0.1%) using glass beads (0.5mm) and vortexing. Following centrifugation to remove unbroken cell debris, monoclonal antibody specific for p55 was added to both extracts and incubated overnight at 4°C. The antibody complex was harvested by the addition of goat anti-mouse $IgG_1$ antibody coupled to SEPHAROSE. The antibody complex removed by the SEPHAROSE was eluted using 0.5M acetic acid, dried in vacuo, analyzed by SDS-PAGE and autoradiographed.

A clear band co-migrating with HPLC-purified gag precursor and an additional 41kDa band were the only labeled species observed. No labeled bands were present in the control lane of gel.

## EXAMPLE 8

### Construction of Insect Expression Vector for HIV Gag Precursor

A baculovirus gene expression system [Summers, M.D. et al., A Manual of Methods for Baculovirus Vectors and Insect Cell Culture Procedures, Texas Agricultural Experiment Station, Bulletin No. 1555, May 1987] was applied to the expression of the HIV gag-pol genes as follows. From plasmid clone P1 of Example 1, the 4.4kb HgaI-NdeI fragment containing the gag-pol genes was excised. The ends of this 4.4kb duplex were "filled-in" with Klenow DNA polymerase, and the following synthetic oligonucleotide duplexes were ligated to the resulting blunt ends:

```
5'-GATCCACAAAACAAAATG-3'
3'-    GTGTTTTGTTTTAC-5'
```

The remaining "sticky" ends thus created constitute BamHI restriction endonuclease recognition sites, while the rest of the ligated sequence includes a yeast translation recognition sequence. The symmetrically linker-modified 4.4kb fragment was subcloned into plasmid AH5, from which it could be recovered as a BamHI fragment. This recovered BamHI fragment was inserted into the unique BamHI site of the baculovirus expression plasmid pAc373 [Summers, M.D, et al., supra]. Clones bearing the gene inserted in the sense orientation relative to the baculovirus polyhedrin promoter in pAc373 were identified by restriction analysis. In addition to the native gag-pol pAc373 expression vector, a second pAc373 clone, in which the gag-pol sequence was modified to contain a fremeshift mutation by Klenow DNA polymerase fill-in of an internal BglII site, was also prepared.

Each of the two vectors was used for separate co-transfections, with Autographa californica Nuclear Polyhedrosis viral DNA, of the Spodoptera frugiperda cell line Sf9 according to the methods of Summers, M.D. et al., supra. Recombinant viral plaques of each vector, wherein the virus has been modified by homologous recombination to contain the gag-pol gene under transcriptional regulation of the late viral polyhedrin promoter, were identified by plaque morphology and the excised agarose plugs (from the plaque

plate) containing the corresponding virus were then eluted overnight in growth medium and used to infect microtiter plate cultures of Sf9 cells. Infected cells, in turn, were assayed for gag expression by immunodot blot with HIV-positive antiserum according to Whang, Y. et al., J. Virol 61, 1796 (1987). Positive viral stocks were "plaque-purified" and re-examined for expression by the Western blot. The native gag-pol viral vector expression system (AcGAG-A-15-3) was found to express a major immunoreactive polypeptide species at ca. 55kDa and a minor species at ca. 40kDa. The frameshift gag-pol viral vector expression system (AcGAG-B-1-3) accumulated primarily a ca. 40kd species. These results are qualitatively similar to results obtained by expression of the same two open reading frames in yeast.

## EXAMPLE 9

### Gag Precursor Sequences

Nucleotide sequencing of pKH12-2 of Example 1 was performed by the dideoxy termination method with modified T7 polymerase. The deduced amino acid sequence is provided in the following Table III.

## TABLE III

```
MGARASVLSGGELDKWEKIRLRPGGKKQYKLKHIVWASRELERFAVNPGLLETSEGCRQILGQLQPS 67
                                                               p17    <> p24
LQTGSEELRSLYNTIAVLYCVHQRIDVKDTKEALDKIEEEQNKSKKKAQQAAADTGNNSQVSQNYPI
VQNLQGQMVHQAISPRTLNAWVKVVEEKAFSPEVIPMFSALSEGATPQDLNTMLNTVGGHQAAMQML
KETLNEEAAEWDRVHPVHAGPIAPGQMREPRGSDIAGTTSTLQEQIGWMTHNPPIPVGEIYKRWIIL
GLNKIVRMYSPTSILDIRQGPKEPFRDYVDRFYKTLRAEQASQEVKNWMTETLLVQNANPDCKTILK
               p24     <                 >p15
ALGPGATLEEMMTACQGVGGPGHKARVLAEAMSQVTNPATIMIQKGNFRNQRKTVKCFNCGKEGHIA
KNCRAPRKKGCWKCGKEGHQMKDCTERQANFLGKIWPSHKGRPGNFLQSRPEPTAPPEESFRFGEET
TTPSQKQEPIDKELYPLASLRSLFGSDPSSQ***
```

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims and its equivalents.

## Claims

1. Yeast recombinant gag precursor of greater than 95% purity or equally pure variant thereof.
2. The gag precursor of Claim 1, wherein said precursor is myristoylated.
3. Insect recombinant gag precursor.
4. The gag precursor of Claim 1 or 2 or 3, of the sequence provided in Table III of Example 9.
5. The gag precursor of Claim 1 or 2 or 3, in combination with any of the antivirals, immunomodulators, antibiotics or vaccines of Table I.
6. The gag precursor of Claim 1 or 2 or 3, in combination with any of the antivirals, immunomodulators, antibiotics or vaccines of Table I, said gag precursor having the sequences provided in Table III of Example 9.

7. An AIDS vaccine comprising greater than 95% pure yeast recombinant gag precursor, or equally purified variant thereof, and a suitable immunological adjuvant, said vaccine capable of producing antibodies specific for gag precursor.

8. Yeast recombinant gag precursor, or variant thereof, useful as a vaccine against AIDS.

9. An AIDS vaccine comprising insect recombinant gag precursor, or variant thereof, and a suitable immunological adjuvant, said vaccine capable of producing antibodies specific for gag precursor.

10. Insect recombinant gag precursor, or variant thereof, useful as a vaccine against AIDS.

11. An AIDS vaccine comprising greater than 95% pure recombinant gag precursor, or equally pure variant thereof, and a suitable vehicle, said vaccine to be used pre- or post-exposure to prevent or treat HIV infection or disease, said vaccine capable of producing antibodies specific for gag precursor.

12. The AIDS vaccine of Claim 11 wherein the recombinant gag precursor is produced in a yeast expression system.

13. The AIDS vaccine of Claim 12 wherein the yeast expression system is U9 #1-5.

14. The AIDS vaccine of Claim 11 wherein the recombinant gag precursor is produced in an insect expression system.

15. The AIDS vaccine of Claim 11 wherein the said vaccine contains one or more determinants for enhancing immunogenicity of gag precursor.

16. The AIDS vaccine of Claim 15 wherein said determinants consist essentially of T-cell amino acid sequences.

17. The AIDS vaccine of Claim 11 wherein said vehicle comprises adjuvant.

18. The AIDS vaccine of Claim 11 wherein said gag precursor or variant thereof is in particulate form or in aggregates.

19. An AIDS vaccine comprising greater than 95% pure yeast recombinant gag precursor in a suitable adjuvant, said gag precursor having been produced from yeast expression system U9 #5-1, said vaccine to be used pre- or post-exposure to prevent or treat HIV infection or disease, said vaccine capable of producing antibodies specific for gag p55 of HIV, serotype New York #5.

20. An AIDS vaccine of Claim 7 or 9 or 11 or 12 or 13 or 14 or 15 or 16 or 17 or 18 or 19, administered in combination with any of the antivirals, immunomodulators, antibiotics or vaccines of Table I.

21. A process of purifying to a purity of greater than 95% the protein yeast recombinant gag precursor, or variant thereof, comprising the steps of

a) thawing a quantity of frozen clarified yeast extract, said yeast extract containing yeast recombinant gag protein precursor, or variant thereof, expressed in a recombinan yeast expression system;

(b) precipitating the gag precursor by freezing for a period of at least about 24 hours;

(c) pelleting by centrifugation the gag precursor precipitate formed, and thereafter separating the pellet from the supernatant;

(d) mixing the pellet with a solution effective for solubilizing membranes and undesired membrane bound proteins;

(e) repelleting the gag precursor by centrifugation to form a second pellet, and thereafter separating the second pellet from the supernatant;

(f) dissolving the pellet of the preceeding step in a solution effective for solubilizing the gag protein presursor, to yield denatured pellet; and

(g) subjecting the denatured pellet to hydrophobic interaction chromatography, and isolating fractions with gag precursor therein, yielding thereby greater than 95% pure yeast recombinant gag precursor or equally pure variant thereof.

22. The process of Claim 21, further comprising additional washing steps between step (e) and step (f) of Claim 21, comprising

(e1) mixing the second pellet with a buffer solution or a solution effective for solubilizing membranes and undesired membrane bound proteins; and

(e2) repelleting the gag precursor by centrifugation to form a third pellet, and thereafter separating the third pellet from the supernatant.

23. The method of rendering water-soluble preparations of yeast recombinant gag precursor or variant thereof, comprising the steps of

(a) taking a portion of substantially pure aggregated gag protein or variant thereof; and

(b) subjecting the protein to reverse phase-high pressure liquid chromatography, yielding substantially pure water-soluble yeast recombinant gag precursor or equally pure water-soluble variant thereof.

24. The process of Claim 21, further comprising the additional step after step (g) of Claim 21, comprising

(h) pooling the fractions of step (g); and

(i) subjecting the pooled fractions to reverse phase-high pressure liquid chromatography, yielding greater than 95% pure water-soluble yeast recombinant gag precursor or equally pure water-soluble variant thereof.

25. The method of rendering water-soluble preparations of yeast recombinant gag precursor or variant thereof, comprising the steps of

(a) taking a portion of substantally pure aggregated gag protein or variant thereof; and

(b) subjecting the protein to gel filtration chromatography in organic phase, yielding substantially pure water-soluble yeast recombinant gag precursor or equally pure water-soluble variant thereof.

26. The process of Claim 21, further comprising the additional step after step (g) of Claim 21, comprising

(h) pooling the fractions of step (g); and

(i) subjecting the pooled fractions to gel filtration chromatography in organic phase, yielding greater than 95% pure water-soluble yeast recombinant gag precursor or equally pure water-soluble variant thereof.

27. Purified yeast recombinant gag precursor with a purity greater than 95%, or equally pure variant thereof, as purified by the process of Claims 21 or 22 or 23 or 24 or 25 or 26.

28. Purified yeast recombinant gag precursor or fragment thereof with a purity greater than 95%, as purified by the process of Claims 21-26, and having the amino acid sequence of Table III in Example 9.

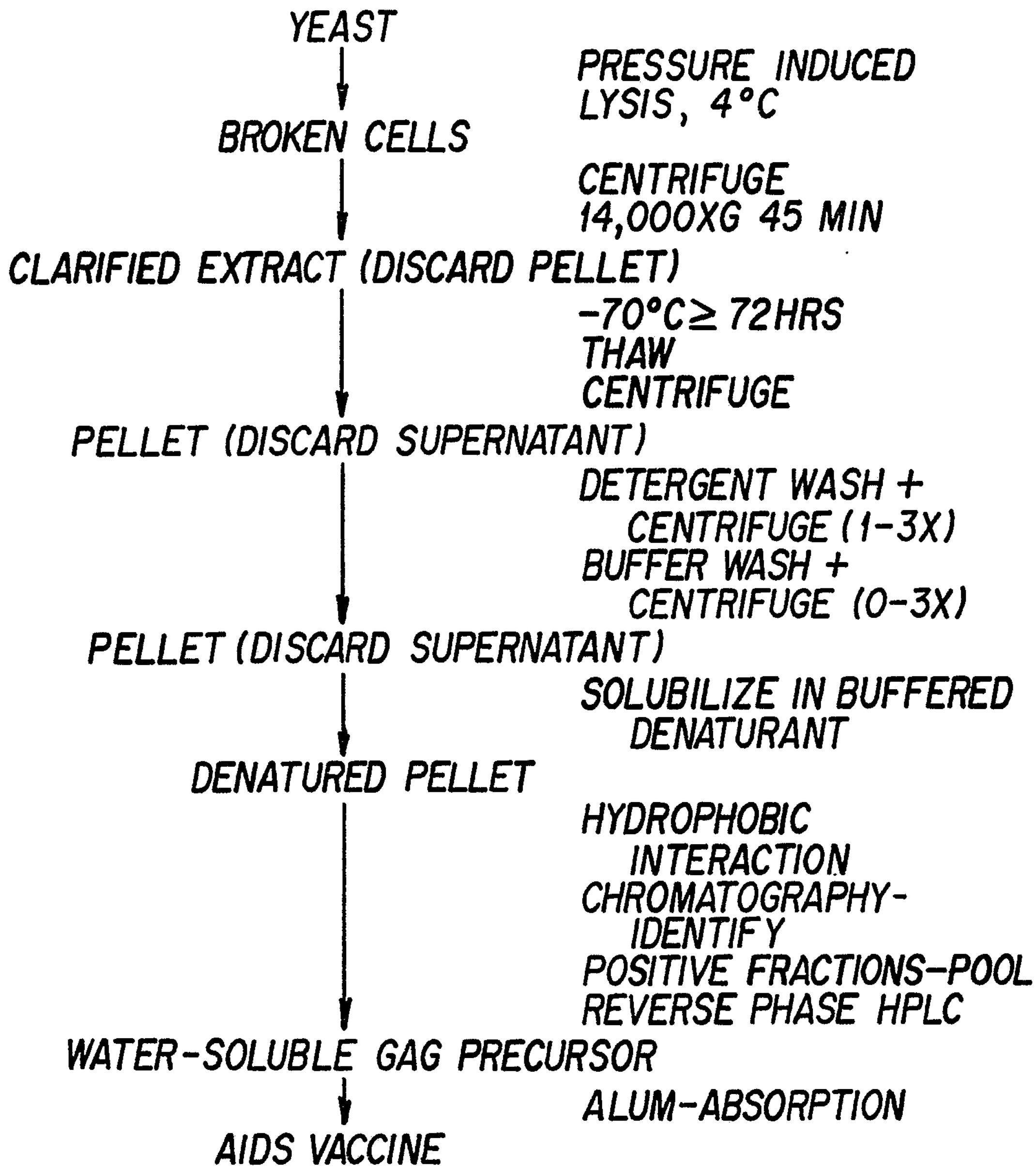
YEAST
PRESSURE INDUCED LYSIS, 4°C
BROKEN CELLS
CENTRIFUGE 14,000XG 45 MIN
CLARIFIED EXTRACT (DISCARD PELLET)
-70°C≥ 72HRS
THAW
CENTRIFUGE
PELLET (DISCARD SUPERNATANT)
DETERGENT WASH + CENTRIFUGE (1-3X)
BUFFER WASH + CENTRIFUGE (0-3X)
PELLET (DISCARD SUPERNATANT)
SOLUBILIZE IN BUFFERED DENATURANT
DENATURED PELLET
HYDROPHOBIC INTERACTION CHROMATOGRAPHY- IDENTIFY
POSITIVE FRACTIONS-POOL
REVERSE PHASE HPLC
WATER-SOLUBLE GAG PRECURSOR
ALUM-ABSORPTION
AIDS VACCINE

FIG. 1

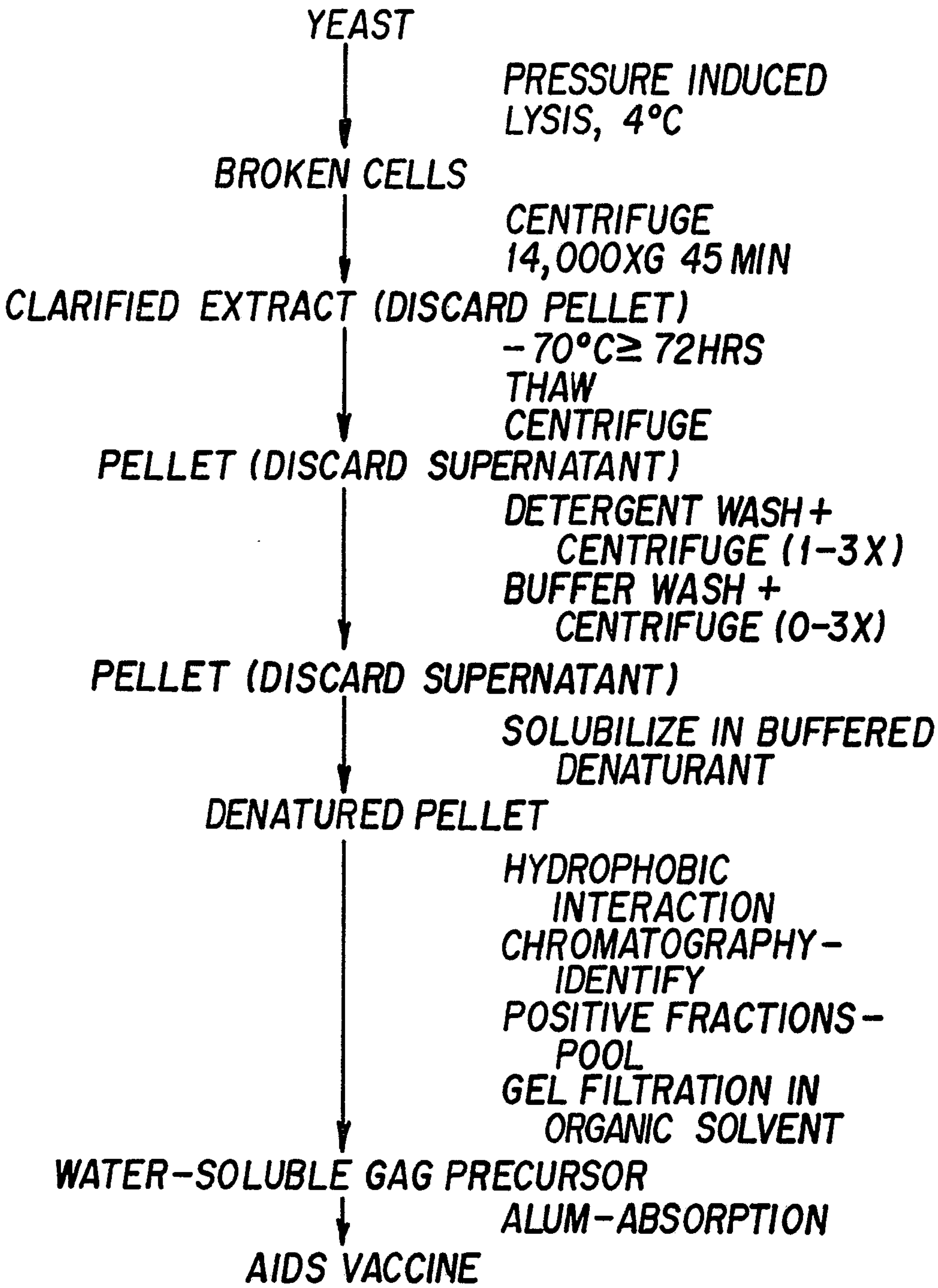
YEAST
PRESSURE INDUCED LYSIS, 4°C
BROKEN CELLS
CENTRIFUGE 14,000XG 45 MIN
CLARIFIED EXTRACT (DISCARD PELLET)
-70°C≥ 72HRS
THAW
CENTRIFUGE
PELLET (DISCARD SUPERNATANT)
DETERGENT WASH + CENTRIFUGE (1-3X)
BUFFER WASH + CENTRIFUGE (0-3X)
PELLET (DISCARD SUPERNATANT)
SOLUBILIZE IN BUFFERED DENATURANT
DENATURED PELLET
HYDROPHOBIC INTERACTION CHROMATOGRAPHY - IDENTIFY POSITIVE FRACTIONS - POOL
GEL FILTRATION IN ORGANIC SOLVENT
WATER-SOLUBLE GAG PRECURSOR
ALUM-ABSORPTION
AIDS VACCINE

FIG. 2

European Patent Office

# EUROPEAN SEARCH REPORT

Application Number

EP 89 20 1037

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 230 222 (F. HOFFMANN LA ROCHE & CO. AG) * pages 3,19,20 * | 1-28 | A 61 K 39/21<br>C 12 N 15/00 |
| X,P | EP-A-0 265 785 (MICROGENESYS INC.) * page 3, line 31 * | 1-28 | |
| X,P | JOURNAL OF VIROLOGY vol. 62, no. 11, November 1988, pages 3993-4002; R. MERVIS et al.: "The gag Gene Products of Human Immunodeficiency Virus Type 1: Alignment within the gag Open Reading Frame, Identification of Posttranslational Modifications, and Evidence for Alternative gag Precursors" * page 3995 * | 1-28 | |
| Y | CHEMICAL ABSTRACTS vol. 99, no. 1, 4 July 1983, page 350; column 1; abstract no. 19402y, Columbus, Ohio, USA; A. SCHULTZ et al.: "In vivo modification of retroviral gag gene-encoded polyproteins by myristic acid" & J. OF VIRO, 1983(46), pages 355-361 * see abstract * | 1-28 | TECHNICAL FIELDS SEARCHED (Int. Cl.4)<br>A 61 K<br>C 12 N |
| Y | CHEMICAL ABSTRACTS vol. 98, no. 9, 28 February 1983, page 222; column 2; abstract no. 67295s, Columbus, Ohio, USA; L. HENDERSON et al.: "Myristyl amino-terminal acylation of murine retrovirus proteins: an unusual post-translational protein modification" & Proc. Natl. Acad. Sci. USA 1983, 80(2), pages 339-343 * see abstract * --- -/- | 1-28 | |

The present search report has been drawn up for all claims

| Place of search | Date of completion of the search | Examiner |
|---|---|---|
| BERLIN | 06-07-1989 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 20 1037

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 603 107 (GENETIC SYSTEMS CORPORATION)<br>* page 4 *<br>--- | 1-28 | |
| A,P | BIOLOGICAL<br>abstract no. 87012267; C. FLEXNER et al.: "Characterization of HIV gag-pol gene products expressed by recombinant vaccina virus" & VIROLOGY 1988, vol. 166, no. 2, pages 339-349<br>----- | 1-28 | |

TECHNICAL FIELDS SEARCHED (Int. Cl.4)

The present search report has been drawn up for all claims

| Place of search | Date of completion of the search | Examiner |
|---|---|---|
| BERLIN | 06-07-1989 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)